# EUROPÄISCHE PATENTANMELDUNG

(11) **EP 1 314 984 A2**
(43) Veröffentlichungstag der Anmeldung: **28.05.2003**
(21) Anmeldenummer: 01250386.8
(22) Anmeldetag: 02.11.2001
(51) Int. Cl.: G01N 33/70, A61K 49/00, A61K 31/195

(54) **Hochkonzentrierte Kreatininlösungen zur Verwendung in Nierenfunktionstests**

(71) Anmelder: Impfstoffwerk Dessau-Tornau GmbH, 06862 Rodleben (DE)
(72) Erfinder: Hartmann, Helmut, Prof.Dr., 12621 Berlin (DE); Höchel, Joachim, Dr., 14548 Caputh (DE)
(74) Vertreter: Müller, Wolfram Hubertus, Dipl.-Phys.

(57) **Zusammenfassung**

Die vorliegende Erfindung betrifft die Verwendung von Kreatinin oder dessen physiologisch annehmbare Salze zur Herstellung hochkonzentrierter pharmazeutischer Zusammensetzungen zur Injektion bei der Untersuchung der Nierenfunktion.

## Beschreibung

Die vorliegende Erfindung betrifft die Verwendung von Kreatinin oder dessen physiologisch annehmbare Salze zur Herstellung hochkonzentrierter pharmazeutischer Zusammensetzungen zur Injektion für die Untersuchung von Nierenfunktionszuständen.

Die Niereninsuffizienz ist insbesondere bei kleinen Haustieren wie Hunden und Katzen nur sehr schwierig zu diagnostizieren. Allerdings ist die Verbreitung dieser Erkrankung nicht unerheblich. Es gibt Untersuchungen, wonach eine chronische Niereninsuffizienz bei etwa 4,2 % der Hunde und 5,6 % der Katzen, welche internistisch erkrankt sind, vorliegt. Bei veterinär-pathologischen Untersuchungen wurde sogar festgestellt, dass 12 % der untersuchten Katzen eine Niereninsuffizienz aufwiesen. Da Heimtiere auch immer älter werden und die Häufigkeit der Niereninsuffizienz mit zunehmendem Lebensalter stark ansteigt, besteht ein großer Bedarf nach geeigneten Untersuchungsverfahren für die Niereninsuffizienz bzw. für die Nierenfunktionsprüfung (Ettinger, S. J., Feldman, E. C., eds. (1995): Textbook of veterinary internal medicine. 4th ed., W. B. Saunders Company Philadelphia and others; Kraft, W., Danckert, D. (1999): Entwicklung einer Katzenpopulation. Teil 1: Anteil der Katze am Patientengut, Geschlechts-, Rassen- und Altersentwicklung - ein Vergleich der Jahre 1967 und 1997. Tierärztl. Prax. 27: 194-197; Kraft, W., Danckert, D. (1999): Entwicklung einer Katzenpopulation - ein Vergleich der Jahre 1967 und 1997. Teil 2: Krankheiten. Tierärztl. Prax. 27: 224-228; Taugner, F., Baatz, G., Nobiling, R. (1996): The renin-angiotensin system in cats with chronic renal failure. J. Comp. Path. 115: 239-252).

Zum Nachweis von Funktionsstörungen der Nieren sind verschiedene Verfahren gebräuchlich. Ein Verfahren stellt die Plasma-Clearance eines iodhaltigen Kontrastmittels dar. Dieses Verfahren ist sehr kostspielig, da ein Analysegerät (z. B. Renalyzer) erforderlich ist. Ein weiteres Verfahren ist die 1-Punkt-Plasma-Clearance von Sinistrin. Dieses Verfahren ist gleichfalls wegen des verwendeten Sinistrins sehr teuer. Weiterhin wird die renale Kreatinin-Clearance in der Klinik verwendet. Dieses Verfahren erfordert eine zeitgenaue Harnsammlung, welche bei kleinen Tieren nur mit großem Aufwand durchführbar ist. Schließlich wird noch die klassische Plasma-Clearance von Kreatinin angewendet. Dieses Verfahren erfordert die Entnahme von 6 - 8 Blutproben im Zeitraum bis zu 12 Stunden.

Diese gebräuchlichen Verfahren sind bei der Untersuchung am Menschen durchaus anwendbar. So ist die Ermittlung der ausgeschiedenen Harnmenge durch Volumenmessung beim Menschen ohne weiteres möglich. Auch die Entnahme von Blutproben über einen längeren Zeitraum ist beim Menschen durchaus ausführbar. Wendet man derartige Verfahren aber bei kleinen Tieren an, so ergeben sich in der praktischen Umsetzung der Verfahren erhebliche Probleme. Harn bei Katzen und Hunden zu sammeln ist nur mittels Katheter möglich. Auch die Entnahme von mehreren Blutproben über einen längeren Zeitraum gestaltet sich in der tierärztlichen Praxis sehr schwierig.

So kommt es, dass derartige Untersuchungen bisher nur in Ausnahmefällen und meist im Rahmen wissenschaftlicher Untersuchungen durchgeführt wurden (Reder, S. und Hartmann, H.; Diagnostische und pathophysiologische Aspekte der Nierenfunktionsprüfung bei Tieren; J. Vet. Med. A. 41, S. 253-267 (1994)).

Labato, M. A. und Ross, L. A. (Plasma disappearance of creatinine as a renal function test in the dog; Research in Veterinary Science, 50, S. 253-258, (1991)) beschreiben die Verwendung einer 5 %-igen Kreatinin-Lösung zur Funktionsdiagnostik bei Hunden. Die Bestimmung der Kreatinin-Clearance wurde dabei mittels eines Blasenkatheters und durch die Entnahme von Blutproben durchgeführt. Dieses Verfahren ist äußerst aufwendig und keinesfalls im Routinebetrieb einer Tierarztpraxis anwendbar.

Es besteht aber ein großer Bedarf an einer Funktionsdianostik für Nierenerkrankungen, welche insbesondere in der Praxis der niedergelassenen Tierärzte einfach und kostengünstig durchführbar ist. An diese Funktionsdiagnostik sind verschiedene Randbedingungen zu stellen. Die verwendete Markersubstanz soll mittels einer einmaligen Injektion verabreicht werden. Die Ermittlung der Plasma-Clearance sollte mit möglichst wenigen Blutproben erfolgen. Die Bestimmung der Markersubstanz im Plasma soll in jedem Routinelabor durchführbar sein. Ferner sollen die Kosten für die Testdurchführung für Tierarzt und Tierhalter akzeptabel sein.

Ein besonderes Problem stellt hierbei die Forderung dar, dass die Markersubstanz mit einer einmaligen Injektion verabreicht werden soll. Für eine zuverlässige Untersuchung der Plasma-Clearance ist die Applikation von ca. 2 g Kreatinin pro m² Körperoberfläche erforderlich. Dies führt zu nicht unerheblichen Injektionsvolumina.

Aufgabe der vorliegenden Erfindung ist es daher, eine Injektionslösung von Kreatinin für die Nierenfunktionsdiagnostik zur Verfügung zu stellen, welche die aufgezeigten Nachteile überwindet.

Die Aufgabe wird durch die Verwendung von Kreatinin oder dessen physiologisch annehmbare Salze zur Herstellung hochkonzentrierter pharmazeutischer Zusammensetzungen zur Injektion für die Untersuchung der Nierenfunktion gelöst.

Überraschenderweise wurde nämlich gefunden, dass im Gegensatz zum Stand der Technik auch hochkonzentrierte Lösungen von Kreatinin in pharmazeutisch und galenisch annehmbarer Qualität herstellbar sind. Dabei bedeutet "hochkonzentriert" im Sinne der Erfindung einen Gehalt von mindestens 9,5 Gew-%.

Erfindungsgemäß bevorzugt ist also die erfindungsgemäße Verwendung, wobei die pharmazeutische Zusammensetzung eine Konzentration an Kreatinin von mehr als 9,5 Gew.-% aufweist.

Ganz besonders erfindungsgemäß bevorzugt ist die erfindungsgemäße Verwendung, wobei die pharmazeutischen Zusammensetzungen eine Konzentration an Kreatinin von mehr als 20 Gew.-% aufweist.

Erfindungsgemäß bevorzugt ist dabei die Verwendung von Kreatinin in Form physiologisch annehmbarer Salze. Hierzu zählen Hydrochlorid, Hydrobromid, Acetat, Formiat, Meleat, Malat, Succinat, Benzoat oder eine Mischung aus mindestens zwei der vorgenannten Salze.

Zum Umfang der vorliegenden Erfindung gehören somit auch pharmazeutische Zubereitungen, welche Kreatinin und/oder dessen physiologisch annehmbare Salze enthalten, wobei deren Konzentration mindestens 9,5 Gew-% beträgt. Besonders bevorzugt sind Lösungen, deren Gehalt mehr als 15 Gew.-% beträgt. Ganz besonders bevorzugt sind Lösungen, deren Gehalt mehr als 20 Gew.-% beträgt.

Erfindungsgemäß besonders vorteilhaft ist es, wenn die Kreatininlösung im Augenblick des Gebrauchs hergestellt wird. Hierzu sind geeignete Mittel an sich bekannt. Hierzu können 2-Kammerspritzen oder lyophilisierte Festsubstanzen verwendet werden, welche dann kurz vor der Injektion zur gebrauchsfertigen Lösung vermischt werden.

Besonders bevorzugt ist es erfindungsgemäß, dass man eine Zweikammerspritze verwendet, um die Lösung herzustellen, wobei in einer Kammer das Kreatinin oder dessen physiologisch annehmbares Salz und in der anderen Kammer das Lösungsmittel vorliegt, wobei diese Komponenten innerhalb der Zweikammerspritze gemischt werden.

Gegenstand der vorliegenden Erfindung ist auch ein diagnostisches Kit, enthaltend eine erfindungsgemäße hochkonzentrierte Kreatininlösung in Form einer oder mehrerer Injektionsampullen, Durchstichflaschen o.ä. bzw. Zweikammerspritzen, Verbrauchsmaterialien zur Blutentnahme und eine Testanleitung nebst Besitzerinformation und gegebenenfalls einen Anforderungsbeleg für die Laborbestimmung und eine Versandtasche für die Proben.

Die erfindungsgemäß hergestellte Kreatininlösung dient zur Funktionsdiagnose von Nierenerkrankungen. Hierzu werden 2 g/m² Kreatinin in Lösung parenteral dem Tier injiziert, nachdem eine Blutprobe zur Bestimmung der endogenen Kreatinin-Konzentration genommen wurde. Im Zeitraum 2 bis 12 Stunden nach Kreatinin-Injektion werden 2 bis 3 Blutproben in angemessenem zeitlichen Abstand entnommen. Auf der Grundlage der gemessenen Kreatinin-Konzentrationen im Plasma dieser Blutproben kann die Ausscheidungsgeschwindigkeit des exogenen Kreatinins berechnet werden. Im Verhältnis zu ermittelten Referenzwerten stellt sie ein Maß der glomerulären Filtrationsrate der Nieren dar.

Die folgenden Beispiele erläutern die Erfindung.

### Beispiel 1 (Herstellung der Lösung)

Zu 23 ml Aqua bidest. werden 2 ml 1 N Salzsäure (HCl) gegeben. Nach Vermischen erfolgt die Zugabe von 2,5 g Kreatinin unter ständigem Rühren. Die Lösung ist klar und enthält eine gemessene Kreatinin-Konzentration von 9,66 mg/dl.

### Beispiel 2 (Nierenfunktionsprüfung bei einer Katze)

Eine Europäische Kurzhaarkatze (männlich, geb. 5/2000, Körpermasse 3,5 kg) hatte eine endogene Kreatinin-Konzentration im Plasma von 530 µmol/l. 3, 4 und 6 Stunden nach Applikation von 4,6 ml einer nach Beispiel 1 hergestellten Kreatininlösung wurden im Plasma Kreatinin-Konzentrationen von 2171, 2028 bzw. 1779 µmol/l gemessen. Die ermittelte Kreatinin-Clearance entspricht damit 32 - 40 % des mittleren Referenzbereiches. Die Filtrationsleistung der Nieren ist hochgradig vermindert.

### Beispiel 3 (Nierenfunktionsprüfung bei einem Hund)

Einem Schäferhund-Mix (weiblich kastriert, geb. 2/1988, 47 kg Körpermasse) wurden 26 ml einer nach Beispiel 1 hergestellten Kreatininlösung i.v. appliziert. Die endogene Kreatinin-Konzentration betrug 1,86 mg/dl. 200, 240 und 300 min nach der Applikation wurden Kreatinin-Konzentrationen von 8,6, 7,65 bzw. 6,7 mg/dl gemessen. Die Kreatinin-Clearance entspricht damit 61 - 71 % des mittleren Referenzbereiches und zeigt eine geringgradig erniedrigte Filtrationsleistung der Nieren an.

## Patentansprüche

1. Verwendung von Kreatinin oder dessen physiologisch annehmbare Salze zur Herstellung hochkonzentrierter pharmazeutischer Zusammensetzungen zur Injektion bei der Untersuchung der Nierenfunktion.

2. Verwendung gemäß Anspruch 1, **dadurch gekennzeichnet, dass** die pharmazeutische Zusammensetzung eine Konzentration an Kreatinin von mehr als 9,5 Gew.-% aufweist.

3. Verwendung gemäß Anspruch 1, **dadurch gekennzeichnet, dass** die pharmazeutische Zusammensetzung eine Konzentration an Kreatinin von mehr als 20 Gew.-% aufweist.

4. Verwendung gemäß Anspruch 1, **dadurch gekennzeichnet, dass** das physiologisch annehmbare Salz Hydrochlorid, Hydrobromid, Acetat, Formiat, Meleat, Malat, Succinat, Benzoat oder eine Mischung aus mindestens zwei der vorgenannten Salze ist.

5. Verwendung gemäß Anspruch 1, **dadurch gekennzeichnet, dass** man die Lösung im Augenblick des Gebrauchs herstellt.

6. Verwendung gemäß Anspruch 5, **dadurch gekennzeichnet, dass** man eine Zweikammerspritze verwendet, um die Lösung herzustellen, wobei in einer Kammer das Kreatinin oder dessen physiologisch annehmbares Salz und in der anderen Kammer das Lösungsmittel vorliegt, wobei diese Komponenten innerhalb der Zweikammerspritze gemischt werden.

7. Diagnostisches Kit, enthaltend eine hochkonzentrierte Kreatininlösung in Form einer oder mehrerer Injektionsampullen, Durchstichflaschen bzw. Zweikammerspritzen, Verbrauchsmaterialien zur Blutentnahme und eine Testanleitung nebst Besitzerinformation und gegebenenfalls einen Anforderungsbeleg für die Laborbestimmung und eine Versandtasche für die Proben.
